(19) Europäisches Patentamt

European Patent Office

Office européen des brevets

(11) EP 1 340 978 A1

(12) EUROPEAN PATENT APPLICATION
published in accordance with Art. 158(3) EPC

(43) Date of publication:
03.09.2003 Bulletin 2003/36

(51) Int Cl.⁷: G01N 33/536

(21) Application number: 01999810.3

(86) International application number:
PCT/JP01/10456

(22) Date of filing: 29.11.2001

(87) International publication number:
WO 02/046755 (13.06.2002 Gazette 2002/24)

(84) Designated Contracting States:
AT BE CH CY DE DK ES FI FR GB GR IE IT LI LU MC NL PT SE TR

(30) Priority: 04.12.2000 JP 2000368973

(71) Applicant: MATSUSHITA ELECTRIC INDUSTRIAL CO., LTD.
Kadoma-shi, Osaka 571-8501 (JP)

(72) Inventors:
• KAWAMURA, Tatsurou
  Kyotanabe-shi, Kyoto 610-0351 (JP)
• KAMEI, Akihito
  Yawata-shi, Kyoto 614-8295 (JP)

(74) Representative:
Leson, Thomas Johannes Alois, Dipl.-Ing.
Patentanwälte
Tiedtke-Bühling-Kinne & Partner,
Bavariaring 4
80336 München (DE)

(54) **METHOD OF DETERMINING SOLUTION CONCENTRATION AND METHOD OF EXAMINING URINE**

(57) To provide a method for measuring an antigen concentration in a solution, which method is highly reliable and practical as well as being labor-saving even in an antigen excess region, an antibody solution is mixed in a sample solution containing an antigen to measure a turbidity, and an acidic solution is further added therein to measure a turbidity, thereby determining the concentration of the antigen from these measured values.

## F I G. 1

## Description

Technical Field

The present invention relates to a method for measuring a concentration of an antigen dissolved in a sample solution, particularly albumin in urine.

Background Art

In a conventional method for measuring a concentration of an antigen contained in a sample solution, an antibody which specifically binds to the antigen is firstly mixed in the sample solution to form an antigen-antibody complex by an antigen-antibody reaction. The sample solution opacifies during the formation, and the antigen concentration is determined based on the turbidity. In this method, measurement has been performed in a region in which an antigen molar concentration is equivalent to an antibody molar concentration (equivalence region), through a region in which the antibody molar concentration is higher than the antigen molar concentration (antibody excess region).

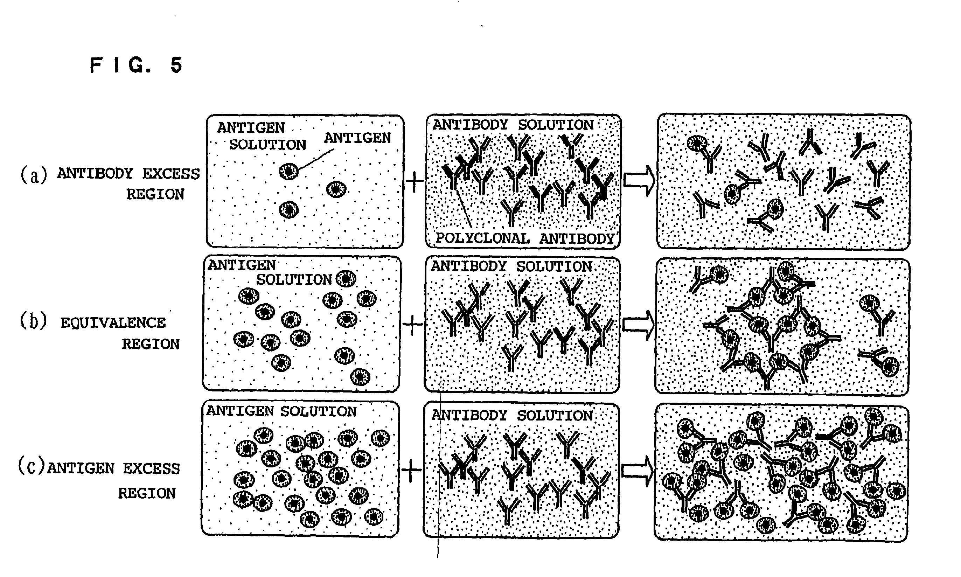

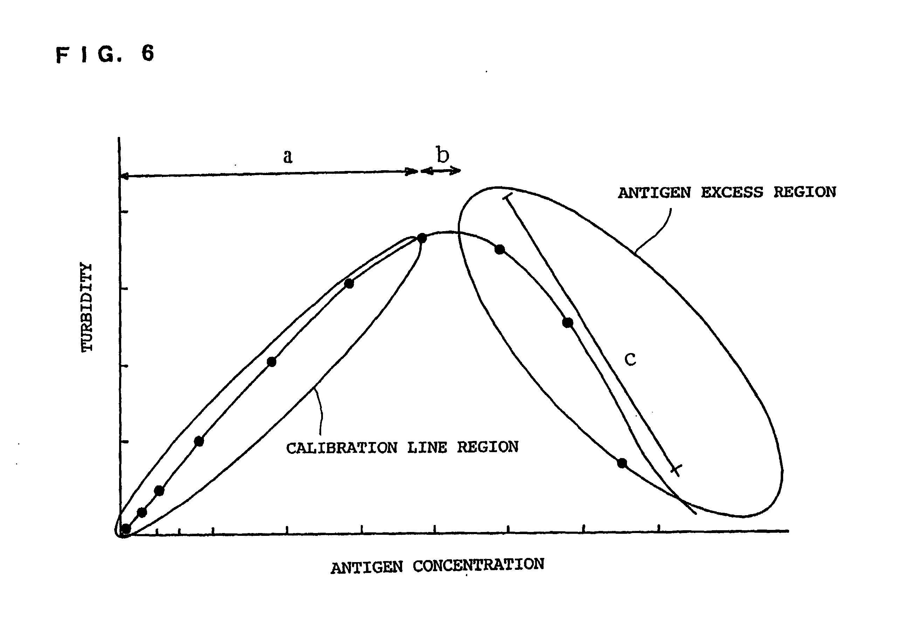

The reason that the measurement has been performed in such regions is described by referring to FIGS. 5 and 6 as well as using a polyclonal antibody as a typical example.

When a solution containing a multivalent antigen having a plurality of antigenic determinants is mixed with a solution containing a divalent antibody having two antigen-binding sites, the antibody and the antigen bind to each other. Herein, FIG. 5 shows how the binding between an antigen and an antibody changes when the antigen molar concentration is increased while the antibody molar concentration is kept constant.

FIG. 5 is a diagram conceptually showing the binding between an antigen and an antibody for the case where the antigen molar concentration is fluctuated while the antibody molar concentration is kept constant. FIG. 5 (a) shows the binding for the case (antibody excess region) where the antigen molar concentration is low and the antibody molar concentration is sufficiently higher than the antigen molar concentration after the mixing.

In a case (equivalence region) where the antigen molar concentration is increased such that the antibody molar concentration is nearly equivalent to the antigen molar concentration after the mixing, the antigen is crosslinked by the antibody to form a large particle, as shown in FIG. 5 (b).

FIG. 5 (c) shows the binding for the case (antigen excess region) where the antigen molar concentration is further increased such that the antibody molar concentration is lower than an equivalence region after the mixing. Herein, a maximum of two antigens bind to a single antibody, so that the antibody molar concentration becomes sufficiently lower than the antigen molar concentration when the antibody molar concentration is not more than about a half of the antigen molar concentration. That is, when the antibody molar concentration is lower than about a half of the antigen molar concentration after the mixing, an antigen excess region is given, and the binding for this case is shown in FIG. 5 (c). Certainly, the standard "half" varies depending on the binding constant between the antigen and the antibody.

Next, FIG. 6 shows the turbidity of a sample solution corresponding to the binding shown in FIG. 5 (a) to (c). As shown in FIG. 6, the turbidity increases with the antigen concentration in the region "a", whereas the turbidity hardly changes with the antigen concentration in the region "b". Then, in the region "c", the turbidity decreases as the antigen concentration increases.

Accordingly, in order to measure an antigen concentration, it is preferable to calculate the antigen concentration from the measured value of turbidity based on a calibration line prepared in the region "a" in FIG. 6.

In this case, it is necessary to set the antibody molar concentration so as to be not less than about a half of the maximum antigen concentration that can be exhibited by the sample solution, more preferably, higher than the antigen molar concentration. In other words, it is necessary to set the antibody molar concentration such that the antigen molar concentration in the sample solution will not be higher than the antibody molar concentration after the mixing. That is, it is necessary to set the antibody molar concentration such that at least the antigen molar concentration in the sample solution will not be about two times or higher than the antibody molar concentration after the mixing. Certainly, the standard "two times" varies depending on, for example, the binding constant between the antigen and the antibody.

As such, since the conventional method for measuring an antigen concentration contained in a sample solution requires the antigen concentration to be measured in an antibody excess region through an equivalence region, it is necessary that the antibody concentration be kept high. In this case, there is a problem that the sensitivity in a low concentration region may be sacrificed.

Moreover, in order to confirm that the sample solution is not indeed in an antigen excess region, i.e., the region "c" in FIG. 6, or to eliminate the antigen excess region, operations such as dilution of the sample solution and further addition of the antibody to the sample solution are performed. This has posed a problem of complicating the steps of the measurement method.

Further, a sample cell, which holds a sample solution during measurement of the turbidity, may be contaminated through repeated use, thereby changing the measured value of the turbidity.

It is an object of the present invention is to provide a method for measuring a solution concentration that is capable of setting an antibody concentration at which the sensitivity in a low concentration is not sacrificed, eliminating the foregoing problems.

It is another object of the present invention to provide a method for measuring a solution concentration that does not necessitate operations such as dilution of the sample solution and further addition of the antibody to confirm the presence or absence of an antigen excess region, i.e., the region "c" in FIG. 6.

It is still another object of the present invention to provide a method for measuring a solution concentration that is capable of correcting errors in a turbidity measurement caused by contamination of the sample cell.

Disclosure of Invention

The present invention relates to a method for measuring a solution concentration characterized by comprising the steps of: (A) mixing in a sample solution, an antibody which binds to a specific antigen in the sample solution in a sample cell; (B) measuring a turbidity of the sample solution after mixing therein the antibody; (C) mixing in the sample solution after mixing therein the antibody, an acidic solution which coagulates a protein component in the sample solution; and (D) measuring a turbidity of the sample solution after mixing therein the acidic solution, the steps (A) to (D) being performed in alphabetical order, wherein an antigen concentration in the sample solution is calculated from the turbidity obtained in the step (B) and the turbidity obtained in the step (D).

In the above-described method for measuring a solution concentration, it is preferable that an amount of the antibody mixed in the step (A) is an amount giving an antigen excess region in a curve showing a relation between a turbidity of and an antigen concentration in the sample solution. In other words, a free antigen may be present in the sample solution after mixing therein the antibody.

For example, it is preferable that the antibody is a divalent antibody having two antigen-binding sites per one molecule, and that an antigen molar concentration is not less than two times an antibody molar concentration in the sample solution in the step (B).

In the above-described method for measuring a solution concentration, it is also preferable that whether or not the antigen is in excess is decided based on a difference between the turbidity obtained in the step (B) and the turbidity obtained in the step (D), and that the antigen concentration in the sample solution calculated from the turbidity obtained in the step (B) is determined.

It is also preferable that, when the antigen concentration in the sample solution calculated from the turbidity obtained in the step (B) and the turbidity obtained in the step (D) is not more than an antigen excess region, contamination of the sample cell is judged based on the turbidity obtained in the step (D).

It is preferable that the acidic solution is a solution of at least one selected from the group consisting of sulfosalicylic acid, trichloroacetic acid, picric acid, tannin, tannic acid and m-galloyl gallic acid.

It is preferable that that a concentration of at least one selected from the group consisting of sulfosalicylic acid, trichloroacetic acid, picric acid, tannin, tannic acid and m-galloyl gallic acid in the sample solution after mixing therein the acidic solution is $5 \times 10^{-3}$ to 5 g/dl.

It is preferable that a pH controlling agent is further added to the sample solution or the acidic solution to adjust a pH of the sample solution to 1.5 to 5.8, in the step (C).

It is preferable that the pH controlling agent is selected from the group consisting of potassium hydrogen phthalate, acetic acid, citric acid and ascorbic acid.

It is also preferable that, when the turbidity obtained in the step (B) is substantially zero, an antigen concentration in the sample solution is calculated from the turbidity obtained in the step (D).

Furthermore, when the sample solution is urine and the antigen is albumin, it is possible to use the above-described method for measuring a solution concentration as a method of urinalysis.

It is preferable that light for use in the turbidity measurement of the sample solution has a wavelength of not shorter than 500 nm.

Further, the method of measuring a solution concentration and/or the method of urinalysis in accordance with the present invention can be carried out by using an apparatus for measuring a solution concentration comprising: a light source for irradiating a sample solution with light; a sample cell for holding the sample solution such that the light transmits through the sample solution; a photosensor 1 and/or a photosensor 2 respectively disposed so as to detect the light which has transmitted through the sample solution and to detect a scattered light which has arisen when the light propagated through the sample solution; a mixer 1 for mixing in the sample solution in the sample cell, an antibody solution containing the above-described antibody; a mixer 2 for mixing in the sample solution in the sample cell, the above acidic solution; a computer for controlling the mixers 1 and 2 and analyzing output signals from the photosensor 1 and/or the photosensor 2, wherein the antigen concentration in the sample solution is measured from the output signals from the photosensor 1 and/or photosensor 2 before and after mixing the antibody solution and the acidic solution.

Brief Description of Drawings

FIG. 1 is a side view schematically showing a structure of a measurement apparatus used in an embodiment of the present invention.

FIG. 2 is a top plan view schematically showing the optical system of the measurement apparatus shown in FIG. 1.

FIG. 3 is a graph showing the relation between the albumin concentration in a sample solution and the turbidity of the sample solution after mixing therein an antibody solution.

FIG. 4 is a graph showing the relation between the albumin concentration in a sample solution and the turbidity of the sample solution after mixing therein either an aqueous tannic acid solution or aqueous sulfosalicylic acid solution.

FIG. 5 is a diagram conceptually illustrating the binding between an antigen and an antibody.

FIG. 6 is a graph showing the states shown in FIG. 5 (a) to (c) as well as the relation between the antigen concentration and turbidity of a sample solution.

FIG. 7 is a graph showing the relation between the antigen concentration in a sample solution and the difference ($T_D$-$T_B$) between turbidity $T_D$ measured in the step (D) and turbidity $T_B$ measured in the step (B).

FIG. 8 is a graph showing the relation between the albumin concentration in a sample solution and the turbidity of the sample solution after mixing therein an aqueous sulfosalicylic acid solution.

Best Mode for Carrying Out the Invention

When sulfosalicylic acid, trichloroacetic acid, picric acid, tannin, tannic acid, m-galloyl gallic acid or the like is added to a solution containing protein, the protein components coagulate to opacify the solution as a whole.

The protein components as mentioned herein include not only antigens such as albumin and globulin, but also antibodies. By mixing a solution containing the above-described acid in the sample solution to measure the turbidity, it is possible to measure the concentration of the protein component in the sample solution.

For example, when urine is used as the sample solution, an acidic solution is mixed therein to coagulate albumin and globulin and thereby altering the optical characteristic (turbidity), and then the concentrations of albumin and globulin in the urine can be determined from the difference between the scattered light intensities measured before and after mixing of the acidic solution ((scattered light intensity measured after mixing of the acidic solution)-(scattered light intensity measured before mixing of the acidic solution)) and/or from the ratio of the transmitted light intensity measured after mixing of the acidic solution to that measured before mixing of the acidic solution ((transmitted light intensity measured after mixing of the acidic solution )/(the transmitted light intensity measured before mixing of the acidic solution)).

It should be noted that tannin as mentioned herein is a general term for complicated aromatic compounds widely distributed in the plant kingdom, having many phenol hydroxyl groups (Dictionary of Chemistry, Tokyo Kagaku Dojin Co., Ltd.), and the molecular weights thereof are from about 600 to 2000 (Encyclopaedia Chimica, Kyoritsu Shuppan Co., Ltd.). Tannic acid is a substance represented by the formula $C_{76}H_{52}O_{46}$ having a CAS Registry Number of 1401-55-4. Additionally, m-galloyl gallic acid is a substance represented by the formula $C_{14}H_{10}O_9$ having a CAS Registry Number of 536-08-3.

When the above-described acidic solution is mixed in the sample solution, the turbidity increases with the protein concentration. That is, the turbidity increases as the protein concentration increases. However, it is also possible to prevent only the antibody from coagulating by adjusting the type and/or concentration of the acid.

On the other hand, when an antibody solution containing a divalent antibody which binds to protein is mixed with the sample solution, the turbidity increases as the protein concentration increases in an antibody excess region through an equivalence region, whereas the turbidity decreases even when the protein concentration increases in an antigen excess region in which the protein concentration is high.

In view of the foregoing, in the present invention, for example, an antibody solution containing a divalent antibody is firstly mixed in a sample solution (the step (A)). As a result, a multivalent antigen and the antibody in the sample solution bind to each other to opacify the sample solution, and turbidity $T_B$ is measured at this time (the step (B)).

In this state, however, there is a possibility that the sample solution is in an antigen excess region in which the antigen molar concentration is sufficiently higher than the antibody molar concentration, and therefore, an acidic solution is further mixed in the sample solution (the step (C)). Then, turbidity $T_D$ of the sample solution is measured after mixing of the acidic solution (the step (D)). At this time, a turbidity is generated, which corresponds to the antigen concentration originally contained in the sample solution.

More specifically, when the antibody does not coagulate by mixing of the acidic solution in the step (C), if the sample solution is in the state of an antigen excess region, the turbidity significantly increases with this antigen concentration upon mixing of the acidic solution. If the sample solution is not in the state of an antigen excess region, the turbidity hardly changes.

Alternatively, when the antibody coagulates by mixing of the acidic solution, the turbidity increases with the antibody concentration, and at the same time, if the sample solution is in the state of an antigen excess region, the turbidity significantly increases with this antigen concentration. If the sample solution is not in the state of an antigen excess region, the turbidity simply increases with the antibody concentration.

In brief, with the turbidity in the step (B) defined as $T_B$ and the turbidity in the step (D) as $T_D$, the above-described phenomena can be summarized as follows.

(1) When the antibody does not coagulate by mixing of the acidic solution,

(i) if the antigen concentration in the sample solution is in an antigen excess region,

$$T_D = T_B + T_{AG}$$

wherein $T_{AG}$ is a turbidity which increases with the antigen concentration;

(ii) if the antigen concentration in the sample solution is in an antibody excess region through an equivalence region,

$$T_D \fallingdotseq T_B$$

(2) When the antibody coagulates by mixing of the acidic solution,

(i) if the antigen concentration in the sample solution is in an antigen excess region,

$$T_D = T_B + T_{AB+AG}$$

wherein $T_{AB+AG}$ is a turbidity which increases with the antibody and antigen concentrations;

(ii) if the antigen concentration in the sample solution is in an antibody excess region through an equivalence region,

$$T_D = T_B + T_{AB}$$

wherein $T_{AB}$ is a turbidity which increases with the antibody concentration.

It should be noted that whether or not the antibody coagulates by mixing of the acidic solution depends upon the type of the acid and/or the concentration of the acid.

Therefore, according to the above (1) and (2), it is possible to ascertain whether or not the sample solution is in an antigen excess region from the coagulating property of the antibody by acids and the amount of change in the turbidity generated after mixing of the acidic solution, while determining the concentration if it is in an antigen excess region.

As such, it is possible to determine the antigen concentration in the sample solution from the turbidity obtained when the antibody solution is mixed in the sample solution and the turbidity obtained when the acidic solution is further mixed therein. As the antigen, one which coagulates by mixing therein the acidic solution, for example, albumin may be employed.

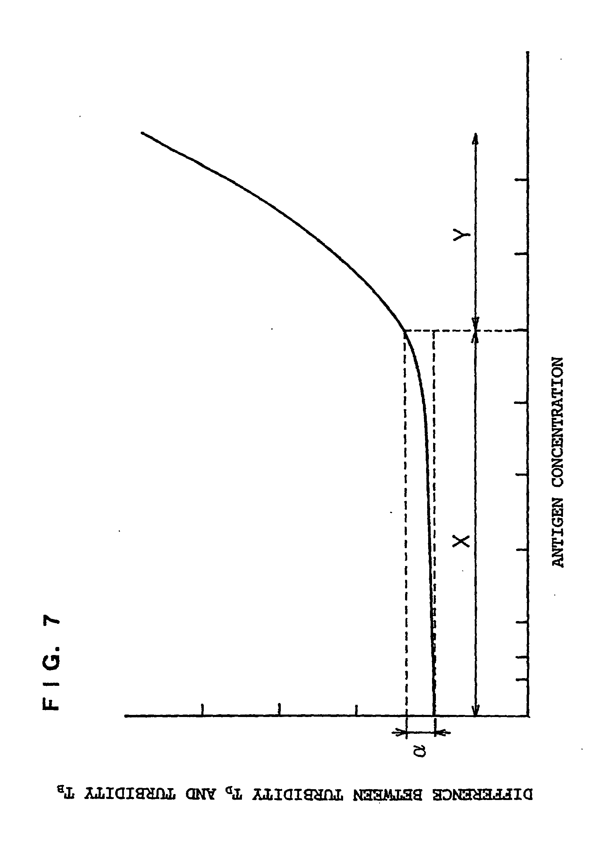

Herein, FIG. 7 shows the relation between the antigen concentration in the sample solution and the difference ($T_D$-$T_B$) between turbidity $T_D$ obtained in the step (D) and turbidity $T_B$ obtained in the step (B) for the case of the above (1) in which the antibody does not coagulate by mixing of the acidic solution in the step (D).

Since the step (B) of mixing the antibody solution has already been performed, $T_B$ is constant. Even when the acidic solution is mixed in the step (C), there are few substances left in the sample solution to coagulate in an antibody excess region through an equivalence region (the portion X in FIG. 7), so that $T_D \fallingdotseq T_B$ holds. Therefore, the calibration line extends substantially in parallel in the portion X shown in FIG. 7; however, when the sample solution is, for example, urine, a free antigen, which is not bound to the antibody, or protein other than the antigen and the antibody may coagulate, so that the turbidity also increases slightly as the antigen concentration increases ($\fallingdotseq T_B + \alpha$). Accordingly, in each case, $T_D$ includes turbidity "$\alpha$" attributed either to the free antigen, which is not bound to the antibody, or to protein other than the antigen and the antibody, in addition to $T_B$, $T_{AG}$, $T_{AB+AG}$ or $T_{AB}$. This "$\alpha$", however, may be ignored depending on the accuracy of the measurement apparatus.

On the other hand, in an antigen excess region (the portion Y in FIG. 7), $T_D$-$T_B$ also increases as $T_D$ increases in the step (D), while the antigen concentration increases.

According to the method for measuring a solution concentration in accordance with the present invention, it is possible to determine a concentration of protein such as albumin contained in: body fluids such as urine, cerebrospinal fluid, blood serum, plasma and saliva; liquid food products such as a dairy product, liquor and vinegar; industrial fluids such as a nutrient solution; fluid used in artificial dialysis and its waste fluid and the like.

In the following, the present invention is described in further detail by way of examples; however, the present invention is not limited thereto.

Example 1

In the present example, urine was used as a sample solution to measure an albumin concentration in the urine. Additionally, an aqueous tannic acid solution was used as an acidic solution. Specifically, an aqueous antibody solution containing a rabbit polyclonal antibody against human albumin was mixed in the sample urine. The antibody concentration and mixing ratio in the aqueous antibody solution were set such that the antibody concentration was about 0.375 mg/ml after this mixing.

Herein, the polyclonal antibody was a divalent antibody and the molecular weight thereof was about 150,000. Accordingly, the antibody molar concentration was $2.5 \times 10^{-6}$ mol/l (2.5 μM) after the mixing. The aqueous tannic acid solution as a reagent had a concentra-

tion of $3\times10^{-3}$ M (mol/L) ($\fallingdotseq0.5$ g/dl), and was mixed in the sample solution at a volume ratio of 1 to 99. Accordingly, the tannic acid concentration was $3\times10^{-5}$ M ($\fallingdotseq5\times10^{-3}$ g/dl) after the mixing.

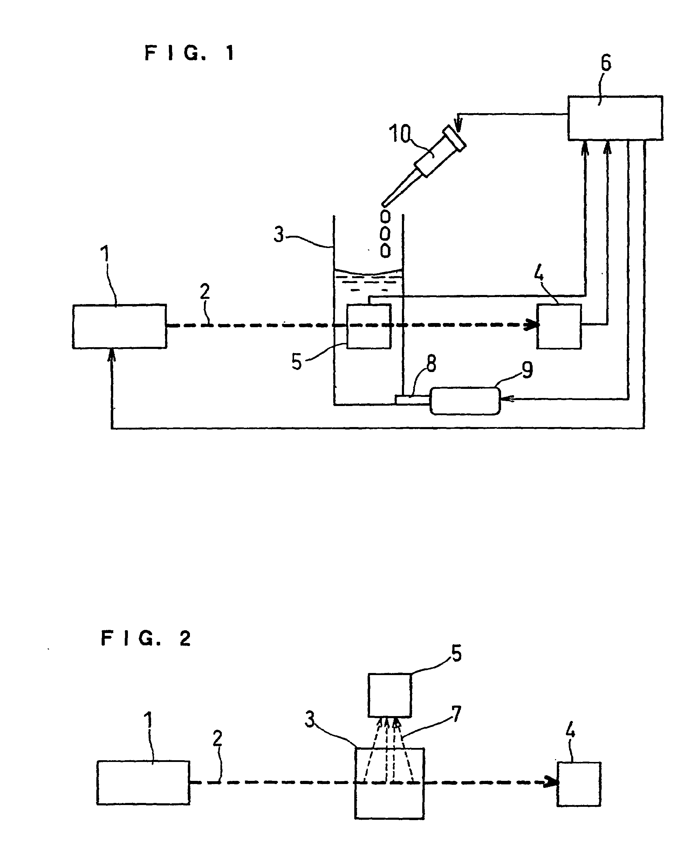

The present example is described by reference to FIGS. 1 to 4. FIG. 1 is a side view schematically showing a structure of an apparatus used for the method for measuring a solution concentration in accordance with the present invention, and FIG. 2 is a top plan view showing the optical system of the apparatus. In these figures, a semiconductor laser module as a light source 1 projects a substantially parallel light 2 having a wavelength of 780 nm, an intensity of 3.0 mW and a beam diameter of 2.0 mm. A sample cell 3 is made of glass and has an opening open upwards. The sample cell 3 is a rectangular container with a base of $10\times10$ mm and height of 50 mm and has transparent optical windows on the sides thereof.

The sample cell 3 allows irradiation of the substantially parallel light 2 on a sample solution held therein as well as permitting taking a transmitted light and a scattered light 7 outside. The transmitted light and the scattered light are detected by a photosensor 4 for detecting a light which has transmitted through the sample solution and a photosensor 5 for detecting the scattered light 7 which has arisen during propagation of the light in the sample solution, respectively. A computer 6 controls the light source 1 and analyzes output signals from the photosensors 4 and 5. Provided at the bottom of the sample cell 3 is an inlet 8, from which an antibody solution is mixed in the sample solution in the sample cell 3. A pipette 9 mixes the antibody solution in the sample solution, and is controlled by the computer 6. Additionally, a pipette 10 mixes the acidic solution in the sample solution in the sample cell 3, and is controlled by the computer 6.

The above-described measurement apparatus was used to measure an albumin concentration in urine. Firstly, 1.485 ml of the sample solution was introduced into the sample cell 3. The computer 6 operated the light source 1 while starting to monitor output signals from photosensors 4 and 5 at the same time. Next, the computer 6 controlled the pipette 9 so as to mix 1.485 ml of the antibody solution from the inlet 8 into the sample cell 3 (the step (A)). The antibody molar concentration in this antibody solution was $5\times10^{-6}$ mol/l (5 $\mu$M); accordingly, the antibody molar concentration was $2.5\times10^{-6}$ mol/l (2.5 $\mu$M) after mixing of the sample solution. The turbidity was determined from the respective output signals from the photosensors 4 and 5 measured before and after mixing of the antibody solution (the step (B)).

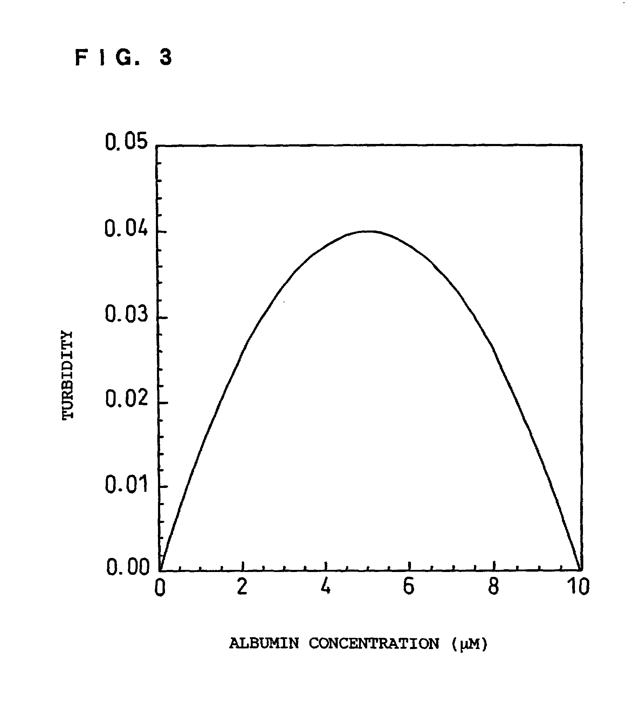

Herein, FIG. 3 shows the relation between the turbidity and the albumin concentration in the sample solution observed after mixing the antibody solution and before mixing the acidic solution. In FIG. 3, the horizontal axis denotes the albumin molar concentration, and the vertical axis denotes the turbidity.

As is clear from this, when the albumin molar concentration was $5\times10^{-6}$ mol/l (5 $\mu$M) or higher, an antigen excess region was obtained and the turbidity decreased as the albumin concentration increased. There were cases where the albumin molar concentration in the urine was $5\times10^{-6}$ mol/l (5 $\mu$M) or higher, and particularly, there was a case where it exceeded $5\times10^{-5}$ mol/l (50 $\mu$M), so that it was not possible to determine the albumin concentration by merely mixing the antibody solution in this manner because an antigen excess region might be present.

Next, as a referential example for the step (C), an aqueous tannic acid solution was mixed in a sample solution.

Herein, an aqueous tannic acid solution was firstly mixed in the sample solution before mixing therein the antibody solution. 2.97 ml of the sample solution was introduced into the sample cell 3, and the computer 6 operated the light source 1, while starting to monitor output signals from the photosensors 4 and 5 at the same time. Next, the computer 6 controlled the pipette 10 so as to mix 0.03 ml of the aqueous tannic acid solution into the sample cell 3. At this time, the concentration of the aqueous tannic acid solution was $3\times10^{-3}$ M ($\fallingdotseq0.5$ g/dl), and the tannic acid concentration was $3\times10^{-5}$ M ($\fallingdotseq5\times10^{-3}$ g/dl) after mixing of the sample solution and the aqueous tannic acid solution. Consequently, the albumin coagulated to opacify the sample solution, decreasing the transmitted light intensity and increasing the scattered light intensity.

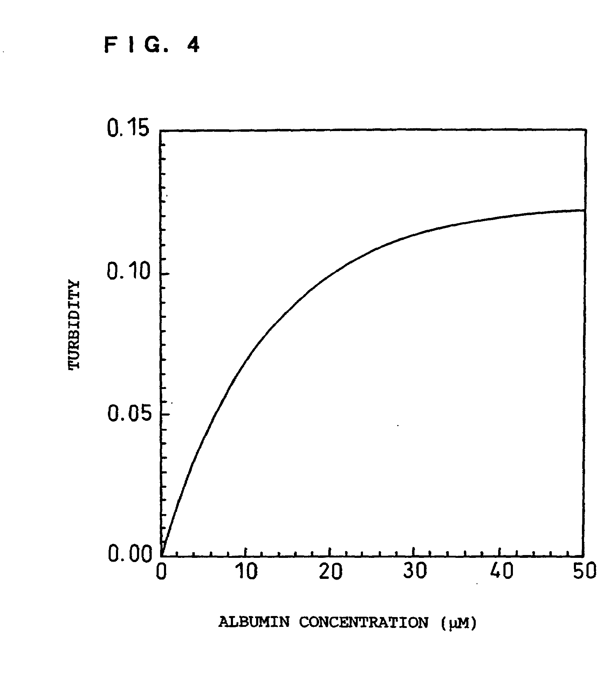

The turbidity was determined from the respective output signals from the photosensors 4 and 5 measured before and after the mixing, and the relation between the turbidity and the albumin concentration in the sample solution was shown in FIG. 4. In FIG. 4, the horizontal axis denotes the albumin molar concentration, and the vertical axis denotes the turbidity. As is evident from this, the turbidity increased as the albumin concentration increased. Accordingly, it was found that the albumin concentration could be determined by measuring the turbidity.

Next, an aqueous tannic acid solution was mixed in the sample solution after mixing therein the antibody solution (the step (C)). At the above-described tannic acid concentration, the antibody did not coagulate, providing the following result.

After the antibody solution was mixed in a sample solution having an albumin molar concentration of about 2 $\mu$M (antibody excess region through equivalence region), the turbidity of the sample solution was 0.025. This was as shown in FIG. 3. Next, the turbidity remained at about 0.025 after a further mixing of the aqueous tannic acid solution.

Similarly, after the antibody solution was mixed in a sample solution having an albumin molar concentration of about 4 $\mu$M, the turbidity was 0.038, and this turbidity remained at about 0.038 even after a further mixing of the aqueous tannic acid solution.

From this, it was found that the turbidity did not change in an antibody excess region through an equivalence region with the measurement accuracy as shown herein.

Additionally, after the antibody solution was mixed in sample solutions having the respective albumin molar concentrations of about 8 μM (antigen excess region) and about 10 μM (antigen excess region), the turbidities were about 0.025 and 0, respectively. After an aqueous tannic acid solution was further mixed in these solutions (the step (C)), the turbidities increased to 0.04 and 0.06, respectively.

From this, it was found that, in an antigen excess region, the turbidity corresponding to the antigen concentration was exhibited after mixing of tannic acid. It should be noted, since the mixing ratio of the aqueous tannic acid solution to the sample solution was 1:99 in the above case, the effect of this dilution was not practically observed as turbidity.

Based on the foregoing, the albumin concentration was determined as follows.

When the turbidity was about 0.02 after mixing of the antibody solution, the albumin molar concentration was expected to be about 1.5 μM or about 8.5 μM, according to FIG. 3. Then, when the turbidity remained at about 0.02 after mixing of the aqueous tannic acid solution, the albumin concentration was determined to be about 1.5 μM. On the other hand, when the turbidity increased, the albumin concentration was determined to be about 8.5 μM.

Additionally, when the turbidity was about 0.0 after mixing of the antibody solution, the albumin molar concentration was expected to be about 0 μM or not less than about 10 μM, according to FIG. 3. Then, when the turbidity remained at 0 after mixing of the antibody solution, the albumin concentration was determined to be about 0 μM. On the other hand, when the turbidity increased to about 0.06, the albumin concentration was determined to be not less than about 10 μM. Herein, when the turbidity increased to about 0.1, the albumin molar concentration was expected to be close to 20 μM.

As described above, according to the present example, it was possible to determine the antigen concentration from the turbidity obtained after mixing of the antibody solution and the turbidity obtained after mixing of the acidic solution.

In the cases of characteristics as shown in FIGS. 3 and 4 in the present example, when the turbidity was not zero after mixing of the antibody solution, it was possible to determine, even in an antigen excess region, the antigen concentration from the turbidity obtained after mixing of the antibody solution, as well as by confirming the turbidity obtained after mixing of the acidic solution.

Further, when the turbidity was zero after mixing of the antibody solution, it was possible to determine the antigen concentration from the turbidity obtained after mixing of the acidic solution. Moreover, when the amount of change in the turbidity was more than a certain amount after mixing of the acidic solution, it was possible to judge that an antigen excess region was obtained.

Next, when an acid was further added to the above acidic solution such that the pH of the sample solution was 1.5 to 5.8 after mixing of the acidic solution in the sample solution, the operation was stable even at a high albumin molar concentration (about not less than 20 μM). As the acid to be added, potassium hydrogen phthalate, acetic acid, citric acid, ascorbic acid and the like particularly provided good stability and reproducibility for the measurement operation.

In the present example, the scattered light intensity measured before mixing of either the antibody solution or the acidic solution, i.e., the difference between the output signals from the photosensor 5 measured before and 300 seconds after the mixing was regarded as the turbidity. The vertical axes in FIGS. 3 and 4 indicate this. This turbidity might be determined based on the transmitted light intensity. For example, the turbidity might be determined from the ratio of the transmitted light intensities measured before and after the mixing.

Furthermore, the turbidity might be determined by using both the scattered light intensity and the transmitted light intensity. In other words, by measuring the both, it was possible to determine a solution concentration of the sample solution in a low concentration region from the scattered light intensity, as well as determining a solution concentration of the sample solution in a high concentration region from the transmitted light intensity, so that an accurately measurable concentration range of the sample solution, that is, a dynamic range, could be expanded. Such improvement in dynamic range is described in detail in JP-A-11-307217.

In this example, the aqueous tannic acid solution reagent had a concentration of $3 \times 10^{-3}$ M ($\fallingdotseq$0.5 g/dl), and was mixed in the sample solution at a volume ratio of 1:99 to adjust the tannic acid concentration to $3 \times 10^{-5}$ M ($\fallingdotseq 5 \times 10^{-3}$ g/dl) after the mixing. It was possible to measure the protein concentration at other tannic acid concentrations after the mixing as long as they were in the range of $3 \times 10^{-5}$ to $3 \times 10^{-2}$ M ($5 \times 10^{-3}$ to 5 g/dl), by forming a calibration line corresponding to each of the tannic acid concentrations obtained after the mixing.

When the tannic acid concentration was lower than the above range, there were cases where the protein did not coagulate, making it difficult to conduct a stable measurement. Alternatively, when the tannic acid concentration was higher than the above range, there were cases where the antibody coagulated to produce a turbidity, or the coagulated protein rapidly precipitated to cause a nonuniform turbidity so that the turbidity did not correspond to the concentration around the region where the substantially parallel light 2 passed, thereby making it difficult to conduct a stable measurement. Accordingly, it was practically preferable to conduct the measurement within the above concentration range.

Further, when the mixing ratio of the sample solution and reagent was different, a different calibration line was obtained, and therefore, it was necessary to form a different calibration line corresponding to the mixing ratio. Also, when tannin and m-galloyl gallic acid were used, a similar operation as described above was possible as long as the concentration was within the range of $5\times10^{-3}$ to 5 g/dl after the mixing.

In the present example, the antibody molar concentration was $2.5\times10^{-6}$ mol/l (2.5 μM) after mixing of the sample solution; however, a similar effect could be achieved at other concentrations. In this case, when the antibody concentration in the sample solution was high after the mixing, the antigen concentration for giving an antigen excess region was also high, naturally. However, when the antibody concentration was high after the mixing, the sensitivity was decreased in a low antigen concentration region.

For example, the albumin concentration in urine might be 5 μM or higher, whereas it rarely exceeded 100 μM. Therefore, it was possible to prevent an antigen excess region (the region "c") from being given even when the antigen concentration was 100 μM, by setting the antibody concentration at about 50 μM. In this case, however, the sensitivity in a low concentration region was sacrificed.

The present invention was particularly effective when the antibody concentration was set such that the sensitivity in a low concentration region was not sacrificed while the concentration could be determined even in an antigen excess region. Specifically, when the antibody was a divalent antibody having two antigen-binding sites per one molecule and the antigen was a multivalent antigen having plural antigenic determinants, it was effective to increase the antibody concentration to such an extent that the antigen molar concentration could be not less than two times the antibody molar concentration after the mixing. In other words, it was effective to set the antibody molar concentration at a molar concentration not more than a half of the maximum antigen molar concentration which could be exhibited by the sample solution.

As described above, according to the present example, it was possible to set an antibody concentration at which the sensitivity in a low concentration region was not sacrificed, and to dispense with steps conventionally required for confirming the absence of an antigen excess region, such as dilution of the sample solution and further addition of the antibody, thereby improving the practical effects for higher accuracy, efficiency and labor saving of the measurement and the test.

In particular, the present invention was practical for measurement of an albumin concentration in urine, since it enabled measurement of the maximum possible albumin concentration of about 100 μM, while ensuring the sensitivity in a low concentration region which was not more than the minimum required concentration of about 1 μM.

Example 2

In the present example, urine was used as a sample solution to measure an albumin concentration in the urine. Additionally, an aqueous sulfosalicylic acid solution with a concentration of 40 g/dl was used as an acidic solution.

Specifically, as in Example 1, an aqueous antibody solution containing a rabbit polyclonal antibody against human albumin was mixed in the sample urine. The antibody concentration was about 0.375 mg/ml (2.5 μM) after the mixing. In addition, the aqueous sulfosalicylic acid solution was mixed in the sample solution at a volume ratio of 1:9. Accordingly, the concentration of sulfosalicylic acid was 4 g/dl after the mixing.

The present example is described by referring to FIGS. 1 to 4. As in Example 1, the present example employed a measurement apparatus as shown in FIGS. 1 and 2. With the use of the measurement apparatus, the albumin concentration was measured in the following manner, using urine as a sample solution.

Firstly, 1.35 ml of the sample solution was introduced into the sample cell 3, and the computer 6 operated the light source 1. Then, at the same time, monitoring of output signals from the photosensors 4 and 5 was started. Next, the computer 6 controlled the pipette 9 to mix 1.35 ml of the antibody solution from the inlet 8 into the sample cell 3 (the step (A)). Since this antibody solution had the antibody molar concentration of $5\times10^{-6}$ mol/l (5 μM), the antibody molar concentration was $2.5\times10^{-6}$ mol/l (2.5 μM) after mixing of the sample solution. The turbidity was determined from the respective output signals from the photosensors 4 and 5 measured before and after the mixing of this antibody solution (the step (B)).

Herein, the relation between the turbidity and the albumin concentration in the sample solution was as shown in FIG. 3 before mixing of the acidic solution, because the antibody concentration obtained after the mixing was the same as that of Example 1. As in Example 1, it was not possible to determine the albumin concentration by merely mixing the antibody solution because there was a possibility that an antigen excess region might be present.

Next, an aqueous sulfosalicylic acid solution was mixed in the sample solution. Herein, the aqueous sulfosalicylic acid solution was firstly mixed in the sample solution before mixing therein the antibody solution. 2.7 ml of the sample solution was introduced into the sample cell 3, and the computer 6 operated the light source 1, while starting to monitor output signals from the photosensors 4 and 5 at the same time. Next, the computer 6 controlled the pipette 10 to mix 0.3 ml of an aqueous sulfosalicylic acid solution to the sample cell 3. At this time, the concentration of the aqueous sulfosalicylic acid solution was 40 g/dl, and the sulfosalicylic acid concentration was 4 g/dl after mixing of the sample solution. Consequently, albumin coagulated to opacify the sample solution, decreasing the transmitted light intensity and increasing the scattered light intensity.

The turbidity was determined from the respective output signals from the photosensors 4 and 5 measured before and after the mixing, and the relation between the turbidity and the albumin concentration in the sample solution was substantially the same as that shown in FIG. 4 of Example 1. Although the present example differed from Example 1 in the mixing ratio of the acidic solution to the sample solution, the difference in the turbidity due to the type of the acid resulted in the same albumin concentration-turbidity characteristics as shown in FIG. 4. As is evident from this, the turbidity increased as the albumin concentration increased. Therefore, it was found that the albumin concentration could be determined by measuring the turbidity.

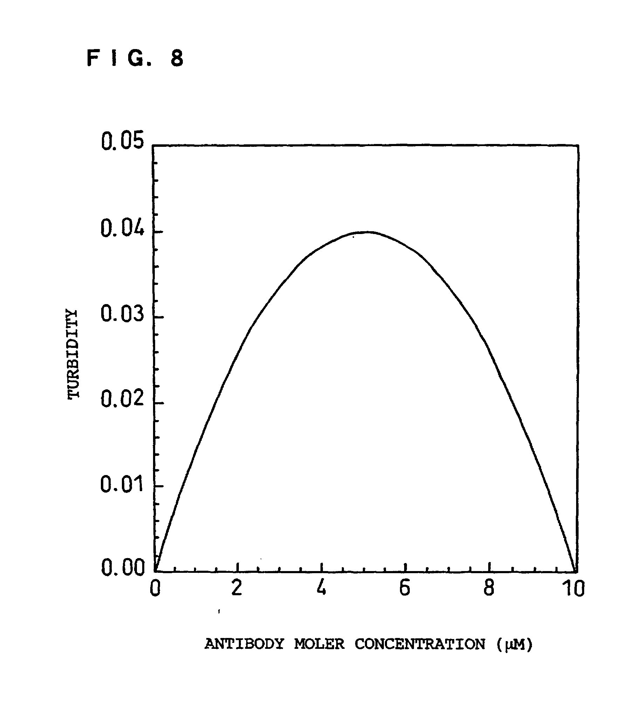

Next, an aqueous sulfosalicylic acid solution was mixed in the sample solution after mixing therein the antibody solution (the step (C)). Although sulfosalicylic acid having the above-described concentration was used, the turbidity of the sample solution corresponded to the antibody concentration, unlike Example 1, even when the antigen coagulated to reduce the albumin concentration to zero. That is, the antibody molar concentration-turbidity characteristics as shown in FIG. 8 were obtained. For example, when the antibody molar concentration was 2.5 µM after the mixing, the turbidity was 0.024.

Further, the turbidity was 0.025 after the antibody solution was mixed in a sample solution having an albumin molar concentration of about 2 µM (antibody excess region through equivalence region). This was as shown in FIG. 3. Next, the turbidity increased to about 0.04 after a further mixing of the aqueous sulfosalicylic acid solution.

Similarly, the turbidity was 0.038 after the antibody solution was mixed in a sample solution having an albumin molar concentration of about 4 µM (antibody excess region through equivalence region), and the turbidity increased to about 0.05 after a further mixing of the aqueous sulfosalicylic acid solution.

From this, it was shown that, in an antibody excess region through an equivalence region, the turbidity increased more when the sulfosalicylic acid solution was mixed than when the antibody solution was mixed, with the measurement accuracy shown herein. However, the increased amount of the turbidity was less than the turbidity obtained when the antibody solution was mixed in the sample solution having an albumin molar concentration of zero and sulfosalicylic acid was further mixed therein, that is, a turbidity of 0.024 of the sample solution containing only the antibody.

Additionally, after the antibody solution was mixed in sample solutions having the respective albumin molar concentrations of about 8 µM (antigen excess region) and about 10 µM (antigen excess region), the turbidities were about 0.025 and 0, respectively. After the aqueous sulfosalicylic acid solution was further mixed in these solutions (the step (C)), the turbidities increased to about 0.06 and 0.07, respectively.

From this, it was found that, even in an antigen excess region, the turbidity increased more after mixing the sulfosalicylic acid than when only the antibody solution was mixed. This increased turbidity was greater than the turbidity obtained when the antibody solution was mixed in the sample solution having an albumin molar concentration of zero and sulfosalicylic acid was further mixed therein, that is, a turbidity of 0.024 of the sample solution containing only the antibody. Furthermore, the turbidity also increased with the antigen concentration.

In an even higher concentration region, with the total molar concentration of the antigen molar concentration and the antibody molar concentration regarded as the molar concentration of the albumin alone, substantially the same turbidities read from FIG. 4 were exhibited. For example, when the albumin molar concentration was 30 µM, the exhibited turbidity was about 0.115. It should be noted that, although the mixing ratio of the aqueous sulfosalicylic acid solution to the sample solution was 1:9 in the above case, the effect of this dilution was not practically observed as a turbidity.

Based on the foregoing, the albumin concentration was determined as follows.

When the turbidity was about 0.02 after mixing of the antibody solution, the albumin molar concentration was expected to be about 1.5 µM or about 8.5 µM, according to FIG. 3. Then, when the turbidity increased after mixing of the aqueous sulfosalicylic acid solution and the increased amount was not more than about 0.024, the albumin concentration was determined to be about 1.5 µM. On the other hand, when the increased amount in the turbidity was not less than 0.024, the albumin concentration was determined to be about 8.5 µM.

Additionally, when the turbidity was about 0.0 after mixing of the antibody solution, the albumin molar concentration was expected to be about 0 µM or about not less than 10 µM, according to FIG. 3. Then, when the turbidity increased after mixing of the antibody solution and the increased amount was not more than about 0.024, the albumin concentration was determined to be 0 µM. On the other hand, the increased amount in the turbidity was not less than 0.024, the albumin concentration was determined to be about 10 µM. Then, when the increased amount in the turbidity was about 0.12, the albumin molar concentration was expected to be close to 30 µM.

As described above, according to the present example, it was possible to determine the antigen concentration from the turbidity obtained after mixing of the antibody solution and the turbidity obtained after mixing of the acidic solution.

In the cases of characteristics as shown in FIGS. 3 and 8 in the present example, when the turbidity was not zero after mixing of the antibody solution, it was possible to determine, even in an antigen excess region, the antigen concentration from the turbidity obtained after mixing of the antibody solution, as well as by confirming the increased amount of the turbidity obtained after mixing of the acidic solution.

Moreover, when the turbidity was zero after mixing of the antibody solution, it was possible to determine the antigen concentration from the turbidity obtained after mixing of the acidic solution.

Further, when the amount of change in the turbidity was more than a certain amount after mixing of the acidic solution, it was possible to judge that an antigen excess region was obtained.

In the present example, the concentration of sulfosalicylic acid was 4 g/dl after the mixing. It was possible to measure the albumin concentration at other concentrations after the mixing, by forming a calibration line corresponding to each of the sulfosalicylic acid concentrations after the mixing. Also, a similar effect could be achieved by employing trichloroacetic acid, picric acid or the like, apart from sulfosalicylic acid.

Additionally, in the present example, the antibody molar concentration was $2.5 \times 10^{-6}$ mol/l (2.5 μM) after mixing of the sample solution; however, a similar effect could be achieved at other concentrations. In this case, when the antibody concentration was high after the mixing, the antigen concentration giving an antigen excess region naturally became high. However, when the antibody concentration was high after the mixing, the sensitivity in a low antigen concentration region was reduced.

For example, the albumin concentration in the urine might be 5 μM or higher, whereas it rarely exceeded 100 μM. Therefore, it was possible to prevent an antigen excess region (the region "c") from being given even when the antigen concentration was 100 μM, by setting the antibody concentration at about 50 μM. In this case, however, the sensitivity in a low concentration region was sacrificed.

The present invention was particularly effective when the antibody concentration was set such that the sensitivity in a low concentration region was not sacrificed while the concentration could be determined even in an antigen excess region. Specifically, when the antibody was a divalent antibody having two antigen-binding sites per one molecule and the antigen was a multivalent antigen having plural antigenic determinants, it was effective to increase the antibody concentration to such an extent that the antigen molar concentration could be not less than two times the antibody molar concentration after the mixing. In other words, it was effective to set the antibody molar concentration at a molar concentration not more than a half of the maximum antigen molar concentration which could be exhibited by the sample solution.

As described above, according to the present example, it was possible to set an antibody concentration at which the sensitivity in a low concentration region was not sacrificed, and to dispense with steps conventionally required for confirming the absence of an antigen excess region, such as dilution of a sample solution and further addition of an antibody, thereby improving the practical effects for higher accuracy, efficiency and labor saving of the measurement and the test.

In particular, the present invention was practical for measurement of an albumin concentration in urine, since it enabled measurement of the maximum possible albumin concentration of about 100 μM, while ensuring the sensitivity in a low concentration region which was not more than the minimum required concentration of about 1 μM.

Moreover, when an albumin concentration determined according to the above was lower than a predetermined value, for example, not more than 0.2 μM, it was possible to correct the concentration by using the turbidity measured after mixing of the acidic solution, or to detect contamination of the sample cell.

In other words, at the antibody concentrations shown in the above-described example, the turbidity would normally be 0.024 after mixing of the aqueous sulfosalicylic acid solution. However, there were cases where the measured turbidity changed owing to, for example, contamination of the sample cell. In this case, the determined albumin concentration was corrected according to the amount or ratio of such change.

For example, when the turbidity was 0.012, which was half the above value, after mixing of the aqueous sulfosalicylic acid solution, the concentration could be calculated from a calibration line after doubling the turbidity measured after mixing of the antibody solution. Also, the concentration could be corrected by using, as a sample solution, a standard solution having an albumin concentration of zero and mixing an antibody solution and an acidic solution in this solution to measure the turbidity, thereby comparing this with a turbidity (in the case where there is no contamination of the sample cell) expected from the antibody concentration obtained after the mixing.

It should be noted that, although the antibody solution was mixed in the sample solution in the sample cell in the above example, a similar effect could be achieved by previously charging the antibody solution in the sample cell and mixing the sample solution therein. However, in this case, the measured value might be influenced by the initial turbidity possessed by the sample solution.

As described above, according to the present invention, it is possible to determine an antigen concentration even in an antigen excess region, thereby realizing a highly reliable, practicable and labor saving measurement of a solution concentration, particularly, measurement of an albumin concentration in urine.

Industrial Applicability

The method for measuring a solution concentration in accordance with the present invention can be suitably applied to a method of urinalysis, by using, as a sample solution, urine which contains albumin as an antigen.

**Claims**

1.  A method for measuring a solution concentration **characterized by** comprising the steps of:

    (A) mixing in a sample solution, an antibody which binds to a specific antigen in said sample solution in a sample cell;
    (B) measuring a turbidity of said sample solution after mixing therein said antibody;
    (C) mixing in said sample solution after mixing therein said antibody, an acidic solution which coagulates a protein component in said sample solution; and
    (D) measuring a turbidity of said sample solution after mixing therein said acidic solution,

    said steps (A) to (D) being performed in alphabetical order,
    wherein an antigen concentration in said sample solution is calculated from the turbidity obtained in said step (B) and the turbidity obtained in said step (D).

2.  The method for measuring a solution concentration in accordance with claim 1, **characterized in that** an amount of said antibody mixed in said step (A) is an amount giving an antigen excess region in a curve showing a relation between a turbidity of and an antigen concentration in said sample solution.

3.  The method for measuring a solution concentration in accordance with claim 2, **characterized in that** said antibody is a divalent antibody having two antigen-binding sites per one molecule, and an antigen molar concentration is not less than two times an antibody molar concentration in said sample solution in said step (B).

4.  The method for measuring a solution concentration in accordance with claim 1, **characterized in that** whether or not said antigen is in excess is decided based on a difference between the turbidity obtained in said step (B) and the turbidity obtained in said step (D), and the antigen concentration in said sample solution calculated from the turbidity obtained in said step (B) is determined.

5.  The method for measuring a solution concentration in accordance with claim 1, **characterized in that**, when the antigen concentration in said sample solution calculated from the turbidity obtained in said step (B) and the turbidity obtained in said step (D) is not more than an antigen excess region, contamination of said sample cell is judged based on the turbidity obtained in said step (D).

6.  The method for measuring a solution concentration in accordance with claim 1, **characterized in that** said acidic solution is a solution of at least one selected from the group consisting of sulfosalicylic acid, trichloroacetic acid, picric acid, tannin, tannic acid and m-galloyl gallic acid.

7.  The method for measuring a solution concentration in accordance with claim 6, **characterized in that** a concentration of at least one selected from the group consisting of sulfosalicylic acid, trichloroacetic acid, picric acid, tannin, tannic acid and m-galloyl gallic acid in said sample solution after mixing therein said acidic solution is $5 \times 10^{-3}$ to 5 g/dl.

8.  The method for measuring a solution concentration in accordance with claim 1, **characterized in that** a pH controlling agent is further added to said sample solution or said acidic solution to adjust a pH of said sample solution to 1.5 to 5.8, in said step (C).

9.  The method for measuring a solution concentration in accordance with claim 8, **characterized in that** said pH controlling agent is selected from the group consisting of potassium hydrogen phthalate, acetic acid, citric acid and ascorbic acid.

10. The method for measuring a solution concentration in accordance with claim 1, **characterized in that**, when the turbidity obtained in said step (B) is substantially zero, an antigen concentration in said sample solution is calculated from the turbidity obtained in said step (D).

11. A method of urinalysis using the method for measuring a solution concentration in accordance with claim 1, **characterized in that** said sample solution is urine and said antigen is albumin.

FIG. 1

FIG. 2

F I G. 3

ALBUMIN CONCENTRATION (μM)

# F I G. 4

F I G. 5

(a) ANTIBODY EXCESS REGION

ANTIGEN SOLUTION    ANTIGEN

ANTIBODY SOLUTION

POLYCLONAL ANTIBODY

(b) EQUIVALENCE REGION

ANTIGEN SOLUTION

ANTIBODY SOLUTION

(c) ANTIGEN EXCESS REGION

ANTIGEN SOLUTION

ANTIBODY SOLUTION

# F I G. 6

EP 1 340 978 A1

# F I G. 7

**F I G. 8**

## INTERNATIONAL SEARCH REPORT

| | |
|---|---|
| International application No. | PCT/JP01/10456 |

**A. CLASSIFICATION OF SUBJECT MATTER**
Int.Cl⁷ $G01N33/536$

According to International Patent Classification (IPC) or to both national classification and IPC

**B. FIELDS SEARCHED**

Minimum documentation searched (classification system followed by classification symbols)
Int.Cl⁷ $G01N33/536$, $G01N33/543$

Documentation searched other than minimum documentation to the extent that such documents are included in the fields searched
Jitsuyo Shinan Koho 1922-1996 Toroku Jitsuyo Shinan Koho 1994-2001
Kokai Jitsuyo Shinan Koho 1971-2001 Jitsuyo Shinan Toroku Koho 1996-2001

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 8-292192 A (Sekisui Chemical Co., Ltd.), 05 November, 1996 (05.11.96) (Family: none) | 1-11 |
| A | JP 4-69572 A (Kabushiki Kaisha Kyoto Ikagaku Kenkyusho), 04 March, 1992 (04.03.92) (Family: none) | 1-11 |
| A | JP 6-118083 A (Godo Shiyusei K.K.), 28 April, 1994 (28.04.94) (Family: none) | 1-11 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 December, 2001 (14.12.01) | 25 December, 2001 (25.12.01) |

| Name and mailing address of the ISA/ | Authorized officer |
|---|---|
| Japanese Patent Office | |
| Facsimile No. | Telephone No. |

Form PCT/ISA/210 (second sheet) (July 1992)